# EUROPEAN PATENT APPLICATION

(11) **EP 1 930 337 A1**
(43) Date of publication of application: **11.06.2008**
(21) Application number: 06796386.8
(22) Date of filing: 11.08.2006
(51) Int. Cl.: C07F 7/04, A61K 9/10, A61K 47/10, C07C 31/20, C07F 7/28, C09K 3/00

(54) **WATER-SOLUBLE METAL ALCOHOLATE DERIVATIVE, PROCESS FOR PRODUCTION OF THE DERIVATIVE, AND SOLID GELATINOUS AGENT FOR EXTERNAL APPLICATION COMPRISING THE DERIVATIVE**

(30) Priority: 12.08.2005 JP 2005234772; 12.08.2005 JP 2005234773
(71) Applicant: SHISEIDO COMPANY, LTD., Tokyo 104-8010 (JP)
(72) Inventor: ISHIKAWA, Yuko, Tsuzuki-ku, Yokohama-shi, Kanagawa, 2248558 (JP); SAKAMOTO, Kazutami, Tsuzuki-ku, Yokohama-shi, Kanagawa, 2248558 (JP); SEKI, Toshihiko, Tsuzuki-ku, Yokohama-shi, Kanagawa, 2248558 (JP); YAJIMA, Isao, Tsuzuki-ku, Yokohama-shi, Kanagawa, 224-8558 (JP); TAKAHASHI, Shun, Tsuzuki-ku, Yokohama-shi, Kanagawa, 224-8558 (JP); WATANABE, Kei, Tsuzuki-ku, Yokohama-shi, Kanagawa, 2248558 (JP)
(74) Representative: Henkel, Feiler & Hänzel
(86) International application number: PCT/JP2006/315948
(87) International publication number: WO 2007/020893

(57) **Abstract**

The object of the present invention is to provide a production method of water-soluble silane derivatives, the separation and purification of which are easy, and the reaction for which can be conducted under an industrially practicable temperature condition (under the temperature condition of 90 °C or lower) and to provide compounds with which solid gelatinous external preparations, with excellent stability against a temperature change and against an effect of additives and with excellent usability, can be prepared. The water-soluble silane derivative could be formed by the substitution reaction of a metal alkoxide with a polyhydric alcohol under the presence of a solid catalyst. According to this production method, the separation of the catalyst from the product is very easy, and the reaction can be carried out under the temperature condition of 90 ° C or lower. The obtained water-soluble metal alcoholate derivative can satisfactory solidify an aqueous formulation of an external preparation by its blending thereinto. The obtained solid gelatinous external preparation has excellent stability against a temperature change and against the effect of additives. In addition, the external preparation is easily disintegrated during use; thus the usability such as easiness to be scooped up with fingers and spreadability during application is excellent.

## Description

### RELATED APPLICATIONS

This application claims the priority of Japanese Patent Application No. 2005-234772 filed on August 12, 2005 and Japanese Patent Application No. 2005-234773 filed on August 12, 2005, which are incorporated herein by reference.

### FIELD OF THE INVENTION

The present invention relates to water-soluble metal alcoholate derivatives, production methods thereof, and solid gelatinous external preparations containing the same, and in particular, relates to the improvement of production methods of water-soluble metal alcoholate derivatives with substituted polyhydric alcohols and the preparation of solid gelatinous external preparations with improved stability and usability.

### BACKGROUND OF THE INVENTION

Silanol groups are formed by the hydrolysis of alkoxy groups in alkoxysilanes; then silica is formed by their subsequent dehydration-condensation. Thus, alkoxysilanes are used in various applications as silica precursors in the sol-gel method. In the past, tetraethoxysilane has widely been used as such an alkoxysilane. However, tetraethoxysilane is insoluble in water, and a hydrolysis reaction does not proceed, as it is, when tetraethoxysilane is added into water. Therefore, tetraethoxysilane was used in an acid or alkaline aqueous solution, and a water-miscible organic solvent such as ethanol was often added as a solubilizing agent. As a result, the addition and removal of acid or alkali and organic solvent was necessary when water-insoluble tetraethoxysilane was used as a precursor for silica. Thus, it was not desirable from the viewpoint of process, cost, and environmental load.

In recent years, polyhydric alcohol substituted silane derivatives have been reported, and these derivatives are very useful for the above-described application because these derivatives are water-soluble compounds. The production of these polyhydric alcohol substituted silane derivatives is usually by the substitution reaction of a tetraalkoxysilane with a polyhydric alcohol, and a homogeneous catalyst (catalyst used after being dissolved in a solvent) such as hydrochloric acid or p-toluene sulfonic acid is used. Thus, there has been a problem in that the catalyst remains in the product and the separation of the product and the catalyst is difficult. A method in which the reaction is carried out under the condition of a high temperature (100 °C or higher) and without a catalyst is also reported. However, this reaction is not practical because the reaction is conducted at a very high temperature. In addition, there is concern that the high-boiling solvent may remain (for example, refer to patent literature 1).

In the base of external preparations such as cosmetics and pharmaceuticals, various thickeners and gelling agents are used to maintain product forms. In the past, as thickeners and gelling agents for the base of water-based products, natural water-soluble polymers such as agar and gelatin or synthetic water-soluble polymers such as polyethylene glycol and acrylic acid polymers were suitably selected according to respective purposes and effects.

In the case of natural water-soluble polymers such as agar, water separation took place at high temperatures, and the stability in a wide temperature range was poor. In addition, there were usability problems in that the easiness to be scooped up with fingers was poor and the spreadability during application was poor. In the case of synthetic water-soluble polymers such as polyethylene glycol, the base could not satisfactorily be solidified because it is a flowable viscous gel. In addition, a decrease in viscosity was significant in the presence of electrolytes and with a pH change. Thus, there was a problem in that the blending components and the production process were limited.

On the other hand, in the case of alkoxysilane such as tetraethoxysilane, it is known that silanol groups are formed by the hydrolysis of alkoxy groups and silica gel is formed by their subsequent dehydration-condensation. However, most of conventionally used alkoxysilanes are insoluble in water, and the hydrolysis reaction does not proceed as it is when an alkoxysilane is added into water. Thus, it is necessary to add other additives; therefore, alkoxysilanes are not suitable for the gelation of an aqueous base. In recent years, a simply mixed aqueous solution of a water-soluble polyhydric alcohol substituted silane derivative was found to form a solid monolithic silica gel. For example, the application to a silica gel precursor for chromatography or the application to a biosensor, on which a biological component such as an enzyme is immobilized, is reported (for example, refer to patent literature 1 and non-patent literatures 1 to 4). However, the use of such a polyhydric alcohol substituted silane derivative has not been tried as an aqueous gelling agent for external preparations.

[Patent literature1] PCT International Publication WO 03/102001
[Non-patent literature1] Sattler et al., Ber. Bunsenges. Phys. Chem., 1998, 102, 1544-1547.
[Non-patent literature2] Mayer et al., J. Phys. Chem. B, 2002, 106, 1528-1533.
[Non-patent literature3] Schipunov, J. Colloid and Interface Sci., 2003, 268, 68-76.
[Non-patent literature4] Schipunov et al., J. Biochem. Biophys. Methods, 2004, 58, 25-38.

### DISCLOSURE OF THE INVENTION

### PROBLEM TO BE SOLVED BY THE INVENTION

The present invention was made in view of the above-described problem of the conventional art, and an object is to provide a production method of water-soluble metal alcoholate derivatives, the separation and purification of which are easy, and the reaction for which can be conducted under an industrially practicable temperature condition (under the temperature condition of 90 °C or lower) and to provide compounds with which solid gelatinous external preparations, with excellent stability against a temperature change and against an effect of additives and with excellent usability, can be prepared.

### MEANS TO SOLVE THE PROBLEM

The present inventors have diligently studied in view of the above-described problem of the conventional art. As a result, the present inventors have found that a water-soluble silane derivative could be formed by the substitution reaction of a metal alkoxide with a polyhydric alcohol under the presence of a solid catalyst. According to this production method, the separation of the catalyst from the product is very easy, and the reaction can be carried out under the temperature condition of 90 °C or lower. The present inventors prepared a water-soluble metal alcoholate derivative with a substituted polyhydric alcohol, blended this into a formulation of a water-based external preparation, and found that it was possible to satisfactorily solidify the base by its hydrolysis in water and the dehydration-condensation reaction. The present inventors also found that the obtained solid gelatinous base had excellent stability against a temperature change and against the effect of additives and that the base is easily disintegrated during use, resulting in the excellent usability such as easiness to be scooped up with fingers and excellent spreadability during application. In addition, the present inventors found that excellent texture, as an external preparation, could be achieved by the formation of mesoscale metal oxide gel and a polyhydric alcohol during the solidification reaction of the water-soluble metal alcoholate derivative in the formulation, thus leading to completion of the present invention.

The water-soluble metal alcoholate derivatives of the present invention are characterized in that they are represented by the following general formula (1).

M¹-(OR¹)ₙ (1)

(In the formula, M¹ represents a Si, Ti, Zr, Zn, or Al atom, and R¹ represents a polyhydric alcohol residue. When M¹ is a Si, Ti or Zr atom, n is 4, when M¹ is a Zn atom n is 2, and when M¹ is an Al atom, n is 3.)

It is desirable that M¹ in formula (1) is Si, or Ti atom in the above-described water-soluble metal alcoholate derivative.
In addition, it is desirable that R¹ in formula (1) is any one selected from the group consisting of an ethylene glycol residue, a propylene glycol residue, a butylene glycol residue, and a glycerin residue in the above-described water-soluble metal alcoholate derivative.

The production method of water-soluble metal alcoholate derivatives of the present invention is characterized in that a metal alkoxide and a polyhydric alcohol are reacted under the presence of a solid catalyst.
It is desirable that the metal alkoxide is titanium alkoxide or alkoxysilane in the production method of the above-described water-soluble metal alcoholate derivatives.
In addition, it is desirable that the reaction is carried out under the temperature condition of 5 to 90 °C in the production method of the above-described water-soluble metal alcoholate derivatives.

In addition, it is desirable that the above-described solid catalyst is an ion exchange resin in the production method of the above-described water-soluble metal alcoholate derivatives.
In addition, it is desirable that the above-described metal alkoxide is tetraethoxysilane in the production method of the above-described water-soluble metal alcoholate derivatives.

In addition, it is desirable that the above-described polyhydric alcohol in the production method of the above-described water-soluble metal alcoholate derivatives is any one selected from the group consisting of ethylene glycol, propylene glycol, butylene glycol, and glycerin.
In addition, it is desirable that the above-described polyhydric alcohol in the production method of the above-described water-soluble metal alcoholate derivatives is any one selected from the group consisting of ethylene glycol, propylene glycol, and butylene glycol and that the reaction is carried out under the temperature condition of 5 to 35 °C.

The solid gelatinous external preparation of the present invention is characterized in that the above-described water-soluble metal alcoholate derivative and water are blended.
It is desirable that a drug component is also blended in the above-described solid gelatinous external preparation.

The production method of the solid gelatinous external preparation of the present invention is characterized in that the above-described water-soluble metal alcoholate derivative is added into a formulation of external preparation containing water.
In addition, the production method of the solid gelatinous external preparation of the present invention is characterized in that the above-described water-soluble metal alcoholate derivative and water are mixed to prepare a solid gel, and the obtained solid gel is added to the formulation of the external preparation.

### EFFECT OF THE INVENTION

According to the production method of the water-soluble metal alcoholate derivative of the present invention, the catalyst separation from a product is easy because the reaction is carried out with the use of a solid catalyst. In addition, the method is highly practicable because the reaction can be carried out under the industrially practicable temperature condition of 90 °C or lower. Thus, the solid gelatinous external preparation that is prepared by blending the obtained water-soluble metal alcoholate derivative into a water-based external preparation formulation has excellent stability against a temperature change and against the effect of additives. In addition, the external preparation is easily disintegrated during use; thus the usability such as easiness to be scooped up with fingers and spreadability during application is excellent. In addition, excellent texture of the external preparation can be achieved during use because a mesoscale metal oxide gel and a polyhydric alcohol are formed during the solidification reaction of the water-soluble metal alcoholate derivative.

### BRIEF DESCRIPTION OF THE DRAWINGS

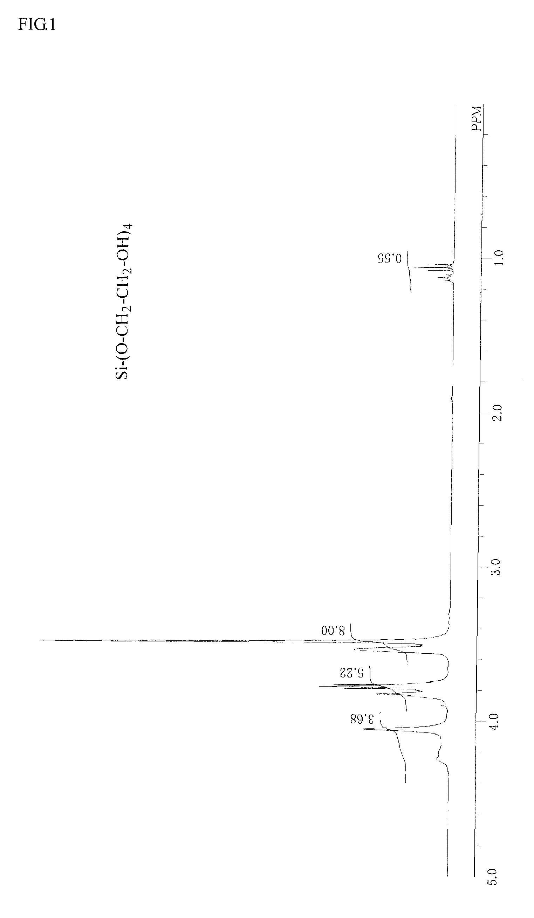

Fig. 1 shows ¹H-NMR results measured for the ethylene glycol substituted silane derivative, (tetra(2-hydroxyethoxy)silane), obtained in Example 1 of the present invention.

### BEST MODE FOR CARRYING OUT THE INVENTION

Water-soluble metal alcoholate derivatives of the present invention are represented by the following general formula (1).

M¹-(OR¹)ₙ (1)

(In the formula, M¹ represents a Si, Ti, Zr, Zn, or Al atom, and R¹ represents a polyhydric alcohol residue. When M¹ is a Si, Ti or Zr atom, n is 4, when M¹ is a Zn atom n is 2, and when M¹ is an Al atom, n is 3.)

In the water-soluble metal alcoholate derivative, which is represented by the above general formula (1) and used in the present invention, M¹ is a Si, Ti, Zr, Zn, or A1 atom. The above-described water-soluble metal alcoholate derivative can be normally prepared by the substitution reaction of a metal alkoxide of monohydric alcohol with a polyhydric alcohol. M¹ varies depending upon what kind of metal alkoxide is used; however, M¹ is preferably a Si or Ti atom from the standpoint of texture of the external preparation during use, and more preferably a Si atom.

In the water-soluble metal alcoholate derivative, which is represented by the above general formula (1) and used in the present invention, R¹ is a polyhydric alcohol residue, and it is represented in a form wherein one hydroxyl group is removed from a polyhydric alcohol. As described above, the water-soluble metal alcoholate derivative can usually be prepared by a substitution reaction of metal alkoxide with a polyhydric alcohol. R¹ varies depending upon what kind of polyhydric alcohol is used. For example, R¹ is -CH₂-CH₂-OH when ethylene glycol is used as the polyhydric alcohol.

Examples of R¹ in the above general formula (1) include an ethylene glycol residue, diethylene glycol residue, triethylene glycol residue, tetraethylene glycol residue, polyethylene glycol residue, propylene glycol residue, dipropylene glycol residue, polypropylene glycol residue, butylene glycol residue, hexylene glycol residue, glycerin residue, diglycerin residue, polyglycerin residue, neopentyl glycol residue, trimethylolpropane residue, pentaerythritol residue, and maltitol residue. Among these, it is preferable that R¹ is any one selected from the group consisting of an ethylene glycol residue, a propylene glycol residue, a butylene glycol residue, and a glycerin residue from the stand point of texture of the external preparation during use.

More specific examples of the water-soluble metal alcoholate derivatives of the present invention include Si-(O-CH₂-CH₂-OH)₄, Si-(O-CH₂-CH₂-CH₂-OH)₄, Si-(O-CH₂-CH₂-CHOH-CH₃)₄, and Si-(O-CH₂-CHOH-CH₂-OH)₄.

The water-soluble metal alcoholate derivative of the present invention can be prepared by the reaction of a metal alkoxide and a polyhydric alcohol under the presence of a solid catalyst.

The metal alkoxide may be any alkoxide so far as alkoxy groups are bonded to any metal atom selected from the group of Si, Ti, Zr, Zn, and Al atoms, and it is not limited in particular. Examples include tetramethoxysilane, tetraethoxysilane, tetraisopropoxysilane, tetrapropoxysilane, tetrabutoxysilane, tetraethoxytitanium, tetramethoxyzirconium, diethoxyzinc, and triethoxyaluminum. Among these, titanium alkoxide or alkoxysilane is preferable, and tetraethoxysilane is especially preferable from the stand point of availability and the safety of reaction by-products.

As an alternative compound for the metal alkoxide, mono-, di-, or trihalogenated metal alkoxide such as monochlorotriethoxysilane, dichlorodimethoxysilane, monobromotriethoxysilane, or tetrachlorosilane could be used. However, these compounds generate strong acids such as hydrogen chloride and hydrogen bromide during the reaction with a polyhydric alcohol. As a result, the corrosion of a reaction apparatus may take place, and the post-reaction separation and removal are difficult; thus they are not practicable.

The polyhydric alcohol may be any so far as two or more of hydroxyl groups are contained in the molecule, and it is not limited in particular. Examples include ethylene glycol, diethylene glycol, triethylene glycol, tetraethylene glycol, polyethylene glycol, propylene glycol, dipropylene glycol, polypropylene glycol, butylene glycol, hexylene glycol, glycerin, diglycerin, polyglycerin, neopentyl glycol, trimethylolpropane, pentaerythritol, and maltitol. Among these, it is preferable to use any one selected from the group of ethylene glycol, propylene glycol, butylene glycol, and glycerin.

The solid catalyst may be any so far as it is a solid catalyst insoluble in raw materials, reaction solvent, and reaction products and it is a solid having active acid points and/or base points for the substituent exchange reaction on the silicon atom. Examples of the solid catalysts used in the present invention include ion exchange resins and various inorganic solid acid/base catalysts.

Examples of ion exchange resins used as the solid catalyst include acidic cation exchange resins and basic anion exchange resins. Examples of matrix resins for these ion exchange resins include styrene-type, acryl-type, and methacryl-type resins. Examples of functional groups with catalyst activity include sulfonic acid, acrylic acid, methacrylic acid, quaternary ammonium, tertiary amine, and primary and secondary polyamines. The matrix structure of ion exchange resin can be selected, depending upon the objectives, from the group consisting of a gel type, a porous type, and a biporous type.

Examples of acidic cation exchange resins include Amberlite IRC76, FPC3500, IRC748, IRB120B Na, IR124 Na, 200CT Na (products of Rohm and Haas Co.), Diaion SK1B, PK208 (products of Mitsubishi Chemical Corporation), DowEX Monosphere 650C, Marathon C, HCR-S, and Marathon MSC (products of Dow Chemical Company). Examples of basic anion exchange resins include Amberlite IRA400J Cl, IRA402BL Cl, IRA410J Cl, IRA411 Cl, IRA458RF Cl, IRA900J Cl, IRA910CT Cl, IRA67, IRA96SB (products of Rohm and Haas Co.), Diaion SA10A, SAF11AL, SAF12A, PAF308L (products of Mitsubishi Chemical Corporation), DowEX Monosphere 550A, Marathon A, Marathon A2, and Marathon MSA (products of Dow Chemical Company).

Inorganic solid acid/base catalysts used as the solid catalyst are not limited in particular. Examples of inorganic solid acid catalysts include single metal oxides such as Al₂O₃, SiO₂, ZrO₂, TiO₂, ZnO, MgO, Cr₂O₃; composite metal oxides such as SiO₂-Al₂O₃, SiO₂-TiO₂, SiO₂-ZrO₂, TiO₂-ZrO₂, ZnO-Al₂O₃, Cr₂O₃-Al₂O₃, SiO₂-MgO, and ZnO-SiO₂; metal sulfates such as NiSO₄ and FeSO₄; metal phosphates such as FePO₄; immobilized sulfuric acid such as H₂SO₄/SiO₂; immobilized phosphoric acid such as H₂PO₄/SiO₂; immobilized boric acid such as H₃BO₃/SiO₂; natural minerals or layered compounds such as activated clay, zeolite, kaolin, and montmorillonite; synthetic zeolite such as AlPO₄-zeolite; and heteropolyacids such as H₃PW₁₂O₄₀·5H₂O and H₃PW₁₂O₄₀. Examples of solid inorganic base catalysts include single metal oxides such as Na₂O, K₂O, Rb₂O, Cs₂O, MgO, CaO, SrO, BaO, La₂O₃, ZrO₃, and ThO₃; metal salts such as Na₂CO₃, K₂CO₃, KHCO₃, KNaCO₃, CaCO₃, SrCO₃, BaCO₃, (NH₄)₂CO₃, Na₂WO₄·2H₂O, and KCN; metal oxide-supported alkali metals such as Na-Al₂O₃ and K-SiO₂; zeolite-supported alkali metals such as Na-mordenite; and composite metal oxides such as SiO₂-MgO, SiO₂-CaO, SiO₂-SrO, SiO₂-ZnO, SiO₂-Al₂O₃, SiO₂-ThO₂, SiO₂-TiO₂, SiO₂-ZrO₂, SiO₂-MoO₃, SiO₂-WO, Al₂O₃-MgO, Al₂O₃-ThO₂, Al₂O₃-TiO₂, Al₂O₃-ZrO₂, ZrO₂-ZnO, ZrO₂-TiO₂, TiO₂-MgO, and ZrO₂-SnO₂.

A solid catalyst can be easily separated, by the treatment such as filtration or decantation, from the result products after the completion of the reaction.

The reaction temperature is not limited in particular, and the reaction is preferably carried out under the temperature condition of 5 to 90 °C. When the reaction is carried out under the temperature condition of higher than 90 °C, there is an issue with the durability of a reaction apparatus. In addition, it is necessary to use a high-boiling solvent for the reaction; as a result, the complete separation and removal of the solvent are difficult. It is preferable to carry out the reaction under the condition of ordinary temperature, namely, under the temperature condition of 5 to 35 °C. When a reaction is carried out under the condition of ordinary temperature, it is desirable to use, as the polyhydric alcohol, any one selected from the group consisting of ethylene glycol, propylene glycol, and butylene glycol. When other polyhydric alcohols such as glycerin are used, reaction products may not be generated under the condition of ordinary temperature.

In the reaction of the present invention, it is not necessary to use a solvent; however, various solvents may be used as necessary. The solvent used in the reaction is not limited in particular, and the examples include aromatic hydrocarbons such as benzene, toluene, and xylene; ester, ether, and ketone solvents such as ethyl acetate, methyl acetate, acetone, methyl ethyl ketone, Cellosolve, diethyl ether, and dioxane; polar solvents such as acetonitrile, dimethylformamide, and dimethyl sulfoxide; and halogen solvents such as chloroform and dichloromethane. In order to suppress the hydrolysis-condensation reaction of a tetraalkoxysilane, which is used as a raw material, it is desirable to dehydrate the solvent in advance. Among these solvents, it is preferable to use acetonitrile, toluene, etc. that can promote the reaction by removing alcohols, such as ethanol, formed as a by-product during the reaction to the outside of the system by forming an azeotropic mixture.

By the above-explained production method, water-soluble metal alcoholate derivatives represented by the following general formula (1) can be obtained.

M¹-(OR¹)ₙ (1)

(In the formula, M¹ represents a Si, Ti, Zr, Zn, or Al atom, and R¹ represents a polyhydric alcohol residue. When M¹ is a Si, Ti or Zr atom, n is 4, when M¹ is a Zn atom n is 2, and when M¹ is an Al atom, n is 3.)

The polyhydric alcohol residue represented by R¹ in the above general formula (1) has a form in which one hydroxyl group is removed from a polyhydric alcohol, such as ethylene glycol, polyethylene glycol, or maltitol. Although R¹ differs depending upon what kind of polyhydric alcohol is used, it is -CH₂-CH₂-OH, when ethylene glycol is used as a polyhydric alcohol.

The terminal -OH groups of the polyhydric alcohol residue R¹ can be connected to the same metal atom or can be connected to the linked metal atoms. For example, they can be water-soluble metal alcoholate derivatives (when n=4) with the structures shown in the following general formulas (2) to (4). By the production method of the present invention, a mixture of these water-soluble metal alcoholate derivatives is obtained.

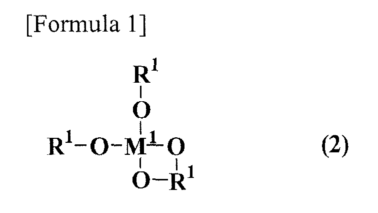

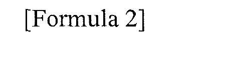

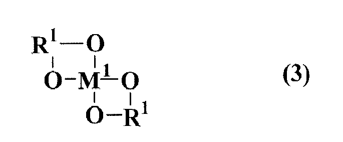

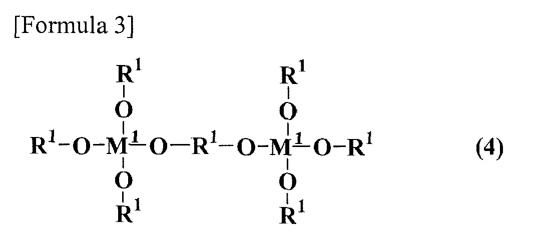

More specifically, the examples of water-soluble metal alcoholate derivatives that can be obtained by the production method of the present invention include Si-(O-CH₂-CH₂-OH)₄, Si-(O-CH₂-CH₂-CH₂-OH)₄, Si-(O-CH₂-CH₂-CHOH-CH₃)₄, and Si-(O-CH₂-CHOH-CH₂-OH)₄.

The water-soluble metal alcoholate derivatives that can be obtained by the production method of the present invention form a mesoscale metal oxide gel (for example, gelatinous silica) and a polyhydric alcohol by the hydrolysis in water and dehydration-condensation reaction. Based on these characteristics, they can be used, for example, as a precursor of porous silica used for chromatography, a silica coating agent of the surface of inorganic or organic compounds, or a forming agent of a silica matrix structure in which a catalyst or enzyme is immobilized. Because the water-soluble metal alcoholate derivatives obtainable by the production method of the present invention can be dissolved in water, it is not necessary to use an acid, alkali, or organic solvent in the hydrolysis and the dehydration-condensation reaction. In addition, a polyhydric alcohol such as ethylene glycol is formed as a by-product of hydrolysis. Thus, the water-soluble metal alcoholate derivatives are very useful, from the standpoint of human body safety, compared with tetraethoxysilane (ethanol is a by-product), which was used for the formation of silica gel in the past.

In the solid gelatinous external preparation of the present invention, two or more selected from the water-soluble metal alcoholate derivatives represented by the above general formula (1) can be combined for blending. The content of the above-described water-soluble metal alcoholate derivatives is not limited in particular so far as the base can be solidified. However, it is preferably 5 to 60 weight % of the total external preparation, and more preferably 10 to 30%. If the content of the above-described water-soluble metal alcoholate derivatives is less than 5 weight %, the system cannot sufficiently be solidified. If the content exceeds 60 weight %, the system becomes too hard and the application may become difficult.

The water-soluble metal alcoholate derivative represented by the above general formula (1) generates a mesoscale metal oxide gel and a polyhydric alcohol in water by hydrolysis and dehydration-condensation reaction. Therefore, in the present invention, the base can easily be solidified by adding the above-described water-soluble metal alcoholate derivative into a water-containing external preparation formulation; thus a solid gelatinous external preparation can easily be prepared. In the present invention, a solid gelatinous external preparation can also be prepared by forming a solid gel beforehand by mixing the above-described water-soluble metal alcoholate derivative and water and then adding the obtained solid gel into an external preparation formulation. In this case, the beforehand prepared solid gel can be added into an external preparation formulation as it is or after disintegrating it into suitable sizes with a publicly known disperser. When the beforehand prepared solid gel is added into an external preparation, water necessary for the solidification of water-soluble metal alcoholate derivative need not to be blended into an external preparation formulation. However, the beforehand prepared solid gel usually contain a large amount of residual water; thus it is desirable to add it into a water-based formulation or as a water-phase component of emulsion formulation

The thus obtained solid gelatinous external preparation of the present invention is superior in the stabilization of the base against a temperature change and against an effect of additives compared with natural or synthetic water-soluble polymers, which have been used in the past as a thickener/gelling agent for a water base. In addition, it is easily disintegrated during use; therefore, the usability such as easiness to be scooped up with fingers and spreadability during application is excellent. The above-described water-soluble metal alcoholate derivative generates a metal oxide gel and a polyhydric alcohol in the formulation during the reaction with water. Therefore, when it is used as a face cleansing scrub, for example, a scrubbing effect due to the metal oxide gel, which is generated during application, can be achieved. In addition, moist feeling after use can be achieved by the moisturizing effect of the polyhydric alcohol, which is widely used as a moisturizing component for cosmetics.

A drug component such as an enzyme is deactivated in the base of a normal external preparation by the effect of the condition or with the elapse of time; thus the retention of activity in the base has been difficult. When such a drug component is blended in the solid gelatinous external preparation of the present invention, the drug component is enclosed in the gel structure. As a result, the long-term retention of activity becomes possible because of structural stabilization. In addition, when a percutaneously absorbable drug is blended in the base of a normal external preparation, the drug dissolves out or the drug is percutaneously absorbed within a short time because of a compositional change after the inunction on the skin; thus it has been difficult to sustain drug efficacy for a long term. When such a drug component is blended in the solid gelatinous external preparation of the present invention, the drug component is enclosed in the gel structure. As a result, the long-term retention of drug efficacy becomes possible because the drug component is gradually released along with the disintegration of the gel structure. Thus, drug components can be suitably blended in the solid gelatinous external preparation of the present invention.

In the solid gelatinous external preparation of the present invention, in addition to the water-soluble metal alcoholate derivative represented by the above general formula (1) and water, which are essential components, other components can be suitably blended, as necessary, so far as the effect of the present invention is not undermined. Examples of other components that can be blended include liquid oil, wax, various surfactants, moisturizers, gelling agents other than the above-described water-soluble metal alcoholate derivatives, water-soluble polymers, lower alcohols, polyhydric alcohols, saccharides, UV absorbers, amino acids, vitamins, drugs, plant extracts, organic acids, organic amines, sequestering agents, antioxidants, antibacterial agents, preservatives, refresheners, perfumes, emollients, pigments, which are normally used as base components or additive components of external preparations such as cosmetics and pharmaceuticals. In addition, whitening agents, anti-wrinkle agents, anti-aging agents, anti-inflammatory agents, hair growth agents, and hair growth-promoting agents, which are used to provide functionality to cosmetics, drugs such as proteases, and drug components for external medicine such as anti-inflammatory agents including steroids and nonsteroids, immunosuppressive agents, analgesic-antiinflammatory agents, antibacterial agents, antifungal agents, antiviral agents, antitumor agents, antiulcer-antidecubitus agents, wound dressing agents, circulation improving agents, antipruritic agents, topical anesthetic agents, anti-motion sickness agents, anti-nicotine agents, and female hormonal agents can also be blended.

In the solid gelatinous external preparation of the present invention, "solid gelatinous" means that the external preparation has no flowability at ordinary temperature. More specifically, it means that the sample does not flow immediately when an external preparation sample is filled in a test tube and the test tube is tilted at 45 ° at ordinary temperature..

The application of the solid gelatinous external preparations of the present invention is not limited in particular so far as the external preparations are used normally for the human body or animals. They can be applied, for example, to cosmetics such as face cleansing scrub, moisturizing scrub, enzyme scrub, foundation pre-makeup, essence, milky lotion, cream, and makeup; drug products containing cataplasm or percutaneously absorbable drug; and various other products.

### EXAMPLES

The present invention will hereinafter be described in further detail by specific examples. However, the present invention is not limited by these examples.

The present inventors initially tried the preparation of ethylene glycol substituted silane derivative with the use of tetraethoxysilane and ethylene glycol under various conditions.

### Ethylene glycol substituted silane derivative

### Comparative Example 1

Tetraethoxysilane (20.8 g (0.1 mol)) and ethylene glycol (24.9 g (0.4 mol)) were mixed with stirring. Under still standing, the mixture was completely separated into two phases. The mixture without solvent and catalyst was vigorously stirred for 24 hours at room temperature; however, no reaction took place.

### Comparative Example 2

To 80 mL of toluene, 20.8 g (0.1 mol) of tetraethoxysilane and 24.9 g (0.4 mol) of ethylene glycol were added. The mixture without a catalyst was refluxed for 24 hours with heating; however, the ethanol azeotrope was not formed and no reaction took place.

### Example 1

To 150 mL of acetonitrile, 20.8 g (0.1 mol) of tetraethoxysilane and 24.9 g (0.4 mol) of ethylene glycol were added, and then 1.8 g of strong acid ion exchange resin (DowEX 50W-X8, a product of Dow Chemical Company) was also added as a solid catalyst. Then the mixture was mixed with stirring at room temperature. The reaction solution, which was initially separated into two phases, became a homogeneous solution after about 1 hour. The stirring of the mixture was continued for 5 days, and then the solid catalyst was separated by filtration. Ethanol and acetonitrile were distilled away under reduced pressure, and 39 g of transparent viscous liquid was obtained. Based on the results of ¹H-NMR analysis, the product was confirmed to be the desired ethylene glycol substituted compound (tetra(2-hydroxyethoxy)silane) (yield: 72.5%). The results of ¹H-NMR analysis of the product are shown in Fig. 1.

### Example 2

Into 50 mL of acetonitrile, 4.19 g (0.02 mol) of tetraethoxysilane and 4.97 g (0.08 mol) of ethylene glycol were added, and then 0.5 g of strong acid ion exchange resin (DowEX 50W-X8, a product of Dow Chemical Company) was also added as a solid catalyst. Then, the mixture was mixed with stirring at room temperature for 48 hours. After the solid catalyst was separated by filtration, ethanol and acetonitrile were distilled away under reduced pressure, and 4.0 g of transparent viscous liquid was obtained. A homogeneous transparent gel was formed by mixing the product with the same amount of water at room temperature (yield: 73%).

### Example 3

Without the presence of solvent, 41.7 g (0.2 mol) of tetraethoxysilane and 49.7 g (0.8 mol) of ethylene glycol were mixed. After adding 1.08 g of strong acid ion exchange resin (DowEX 50W-X8, a product of Dow Chemical Company) as a solid catalyst, the mixture was mixed with stirring at 85 °C. The reaction solution initially separated into two phases and formed an emulsion by stirring. Subsequently, however, the reaction solution became a homogeneous transparent solution. The reaction was continued for 4 hours, and then the solid catalyst was separated by filtration. After the solution was allowed to stand overnight at room temperature, ethanol was distilled away under reduced pressure, and 47.6 g of transparent viscous liquid was obtained. A homogeneous transparent gel was formed by mixing the product with the same amount of water at room temperature (yield: 87%).

### Example 4

Without the presence of solvent, 41.7 g (0.2 mol) of tetraethoxysilane and 49.7 g (0.8 mol) of ethylene glycol were mixed. After adding 1 g of strong acid ion exchange resin (Amberlite CG-120, a product of Rohm and Haas Co.) as a solid catalyst, the mixture was mixed with stirring at 85 °C. The reaction solution initially separated into two phases and formed an emulsion by stirring. Subsequently, however, the reaction solution became a homogeneous transparent solution. The reaction was continued for 4 hours, and then the solid catalyst was separated by filtration. After the solution was allowed to stand overnight at room temperature, ethanol was distilled away under reduced pressure, and 47.6 g of transparent viscous liquid was obtained. A homogeneous transparent gel was formed by mixing the product with the same amount of water at room temperature (yield: 87%).

As shown in Comparative Examples 1 and 2, when a catalyst was not used, no substitution reaction took place either at ordinary temperature or under the conditions of heating, and ethylene glycol substituted silane derivative could not be obtained.
On the other hand, in Examples 1 to 4, wherein an ion exchange resin was used as a solid catalyst, a high yield of ethylene glycol substituted compound could be obtained. In addition, the solid catalyst could easily be separated by filtration after the completion of the reaction. It was also confirmed in Examples 1 and 2 that the ethylene glycol substituted silane derivative could be prepared even under the conditions of ordinary temperature.

Subsequently, the present inventors similarly tried the preparation of polyhydric alcohol substituted silane derivatives using various polyhydric alcohols (propylene glycol, 1,3-butylene glycol, and glycerin).

### Propylene glycol substituted silane derivative

### Comparative Example 3

Into 100 mL of toluene, 11.7 g (0.085 mol) of tetraethoxysilane and 12.09 g (0.16 mol) of propylene glycol were added and stirred. Under still standing, the mixture was completely separated into two phases. The mixture without solvent and catalyst was vigorously stirred under reflux by heating for 24 hours; however, no reaction took place.

### Example 5

Into 100 mL of acetonitrile, 11.7 g (0.056 mol) of tetraethoxysilane and 12.01 g (0.16 mol, 2.9 times in moles with respect to TEOS) of propylene glycol were added, and homogeneous transparent solution was obtained. To this solution, 0.8 g of strong acid ion exchange resin (DowEX 50W-X8, a product of Dow Chemical Company) was added as a solid catalyst. Then the mixture was mixed with stirring for 30 hours at room temperature. The solid catalyst was separated by filtration, ethanol and acetonitrile were distilled away under reduced pressure, and 14.5 g of transparent caramel-like material was obtained. A homogeneous transparent gel was formed by mixing the product with the same amount of water at room temperature (yield: 86%).

### Example 6

Into 20 mL of acetonitrile, 20.8 g (0.1 mol) of tetraethoxysilane and 30.6 g (0.4 mol) of propylene glycol were added, and homogeneous transparent solution was obtained. To this solution, 1.0 g of strong acid ion exchange resin (DowEX 50W-X8, a product of Dow Chemical Company) was added as a solid catalyst. Then the mixture was mixed with stirring for 75 hours at room temperature. The solid catalyst was separated by filtration, ethanol and acetonitrile were distilled away under reduced pressure, and 33.2 g of transparent viscous liquid was obtained. A homogeneous transparent gel was formed by mixing the product with the same amount of water at room temperature (yield: 99%).

### Example 7

To 102.8 g of acetonitrile, 1.03 g of strong acid ion exchange resin (DowEX 50W-X8, a product of Dow Chemical Company) was added as a solid catalyst. Into this, 41.8 g (0.2 mol) of tetraethoxysilane was dissolved, and 59.8 g (0.8 mol) of propylene glycol was dropwise added during a span of about 2 hours with stirring at room temperature. Then the mixture was mixed with stirring for 1 hour at room temperature. The obtained transparent solution was allowed to stand overnight at room temperature, and the solid catalyst was separated by filtration. Ethanol and acetonitrile were distilled away under reduced pressure, and 69.0 g of transparent viscous liquid was obtained. A homogeneous transparent gel was formed by mixing the product with the same amount of water at room temperature (the yield was higher than the theoretical yield 65 g because the residual solvent was contained).

### Example 8

To 120.3 g (1.6 mol) of propylene glycol was added 83.2 g (0.4 mol) of tetraethoxysilane. To this, 2.0 g of strong acid ion exchange resin (DowEX 50W-X8, a product of Dow Chemical Company) was added as a solid catalyst. The mixture without a solvent was mixed with stirring for about 5 hours at room temperature. The obtained transparent solution was allowed to stand overnight at room temperature, and the solid catalyst was separated by filtration. Ethanol was distilled away under reduced pressure, and 140 g of transparent viscous liquid was obtained. A homogeneous transparent gel was formed by mixing the product with the same amount of water at room temperature (the yield was higher than the theoretical yield 130 g because the residual solvent was contained).

### 1,3-Butylene glycol substituted silane derivative

### Example 9

To 55 g of acetonitrile, 1.1 g of strong acid ion exchange resin (DowEX 50W-X8, a product of Dow Chemical Company) was added as a solid catalyst, and 20.8 g (0.1 mol) of tetraethoxysilane was dissolved. To this, 36.2 g (0.4 mol) of 1,3-butylene glycol was added, and the mixture was mixed with stirring for 75 hours at room temperature. After about 5 hours, the solution became slightly cloudy, and a slight increase in viscosity was observed. After the reaction was carried out for 75 hours, the solid catalyst was separated by filtration. Subsequently, ethanol and acetonitrile were distilled away under reduced pressure, and 37.7 g of transparent mobile liquid was obtained. A homogeneous transparent gel was formed by mixing the product with the same amount of water at room temperature (yield: 97%).

### Example 10

To 80 g of acetonitrile, 0.5 g of strong acid ion exchange resin (DowEX 50W-X8: a product of Dow Chemical Company) was added as a solid catalyst, and 72.2 g (0.8 mol) of 1,3-butylene glycol was dissolved. On this occasion, some endotherm was observed. To this, 41.7 g (0.2 mol) of tetraethoxysilane and 30 g of acetonitrile were added, and the mixture was mixed with stirring for 50 hours at room temperature. After the reaction, solid catalyst was separated by filtration. Subsequently, ethanol and acetonitrile were distilled away under reduced pressure, and 75.6 g of transparent mobile liquid was obtained. A homogeneous transparent gel was formed by mixing the product with the same amount of water at room temperature (yield: 98%).

As shown in Examples 5 to 10, when ethylene glycol, propylene glycol, or butylene glycol was used as the polyhydric alcohol, a high yield of polyhydric alcohol substituted silane derivative could be obtained with the use of a solid catalyst. In addition, the solid catalyst could easily be separated by filtration after the completion of the reaction. In addition, it was clarified that these various water-soluble polyhydric alcohol substituted silane derivatives could be prepared even under the conditions of ordinary temperature.

### Glycerin substituted silane derivative

### Example 11

To 100 g of acetonitrile, 36.9 g (0.4 mol) of glycerin was added, and then 0.6 g of strong acid ion exchange resin (DowEX 50W-X8: a product of Dow Chemical Company) was added as a solid catalyst and then mixed. Subsequently, 20.9 g (0.1 mol) of tetraethoxysilane was added, and the mixture was stirred at room temperature. The solution separated into two phases and became cloudy by vigorous stirring. No change was observed even after overnight mixing with stirring at room temperature. The temperature of an oil bath was set at 85 °C, and the mixture was stirred for an additional 48 hours under reflux with heating. After cooling, solid catalyst was separated by filtration. Subsequently, ethanol and acetonitrile were distilled away under reduced pressure; then the product separated into two phases. The two phases were separated and allowed to stand still; then the product of the upper phase separated into a small amount of transparent liquid and a white pasty liquid (6.6 g). The yield of the transparent viscous liquid of the lower phase was 22.9 g. The transparent liquid that was separated from the upper phase neither dissolved into water nor formed a gel. On the other hand, the white pasty product from the upper phase and the transparent viscous liquid from the lower phase formed a homogeneous transparent gel by mixing with the same amount of water at room temperature (yield of the white pasty product and the lower phase product: 75%).

### Example 12

Tetraethoxysilane (60.1 g (0.28 mol)) and glycerin (106.33 g (1.16 mol)) were mixed, and 1.1 g of strong acid ion exchange resin (DowEX 50W-X8, a product of Dow Chemical Company) was added as a solid catalyst. This mixture without a solvent was mixed with stirring at 85 °C. After about 3 hours, the mixture became a homogeneous transparent solution. After the reaction was continued for an additional 5 hours 30 minutes, the obtained solution was allowed to stand overnight. Under reduced pressure, the solid catalyst was separated by filtration and washed with a small amount of ethanol. From this solution, ethanol was distilled away, and 112 g of transparent viscous liquid was obtained. The product formed a homogeneous transparent gel, with a slight exotherm, by mixing with the same amount of water at room temperature (yield: 97%).

As shown in Examples 11 and 12, when glycerin was used as the polyhydric alcohol, a high yield of glycerin substituted silane derivative could be obtained with the use of a solid catalyst. In addition, the solid catalyst could easily be separated by filtration after the completion of the reaction.

### Ethylene glycol substituted titanium alkoxide derivative

### Example 13

A white precipitate was obtained by mixing 14.21 g (0.05 mol) of tetraisopropyl titanate and 12.42 g (0.2 mol) of ethylene glycol without a solvent under a nitrogen atmosphere. The supernatant was measured with NMR, and it was confirmed to be isopropanol. When the obtained white precipitate was added into chloroform, it became a transparent homogeneous solution. When this chloroform solution was dropwise added into water, a white precipitate was generated. This precipitate was dried under reduced pressure and measured with an X-ray diffraction apparatus; as a result, it was confirmed to be titanium oxide.

As shown in Example 13, when titanium alkoxide is used as the metal alkoxide, an ethylene glycol substituted titanium alkoxide derivative could be obtained, in the same way as the above-described silane derivative, with the use of a solid catalyst.

### Solubility of polyhydric alcohol substituted silane derivative

Subsequently, the present inventors evaluated the solubility of the thus obtained polyhydric alcohol substituted silane derivatives in various solvents. The results are shown in Table 1.
The evaluation of solubility was conducted by adding a polyhydric alcohol substituted silane derivative into various solvents so that the concentration would be 5 weight %, mixing and stirring at room temperature, allowing them to stand still, and visually observing the solution state. The evaluation criteria were as follows.
○: completely soluble
Δ: partially soluble
× : insoluble

**[Table 1]**

| | Water | Chloroform | Acetonitrile | Acetone | Ethyl acetate | n-Hexane |
|---|---|---|---|---|---|---|
| Ethylene glycol substituted silane derivative | ○ | ○ | × | × | × | × |
| Propylene glycol substituted silane derivative | ○ | ○ | ○ | ○ | ○ | × |
| 1,3-Butylene glycol substituted silane derivative | ○ | ○ | ○ | ○ | ○ | × |
| Glycerin substituted silane derivative | ○ | Δ | × | × | × | × |

From Table 1, it was confirmed that all the obtained polyhydric alcohol substituted silane derivatives of the present invention had excellent solubility in water. In addition, it was clarified that the propylene glycol substituted compound and 1,3-butylene glycol substituted compound had excellent solubility in other organic solvents.

### Gelation ability of polyhydric alcohol substituted silane derivatives

In order to investigate the gelation ability of the above-described polyhydric alcohol substituted silane derivatives in water, the present inventors visually evaluated the progress of gelation for the 1,3-butylene glycol substituted compound and glycerin substituted compound.
The gelation ability was evaluated by adding the polyhydric alcohol substituted silane derivative to water or KGM medium to obtain various concentrations, mixing and stirring at room temperature, allowing the solution to stand still, and visually observing the gelation. The results are shown in Tables 2 and 3.

**[Table 2]**

| • In aqueous solution | | |
|---|---|---|
| | Concentration % (wt/vol) | Appearance |
| 1,3-Butylene glycol substituted silane derivative | 10.0 | Colloidal turbidity, slight thickening after about 1 week |
| | 20.0 | Colloidal turbidity, thickening after 2 days, translucent low-viscosity gel |
| | 30.0 | Colloidal turbidity, gelation after 2 days, seeping-out liquid on the translucent gel |
| Glycerin substituted silane derivative | 10.0 | Transparent, thickening after about 1 week, agar-like soft gel |
| | 20.0 | Translucent low-viscosity gel after 1 day, some seeping-out ringing gel |
| | 30.0 | Gelation after 1 day, some seeping-out ringing gel |

**[Table 3]**

| • In KGM medium | | |
|---|---|---|
| | Concentration % (wt/vol) | Appearance |
| 1,3-Butylene glycol substituted silane derivative | 2.9 | Transparent solution, slight thickening after 2 hours, pasty after 6 hours (no gelation) |
| | 5.3 | Transparent solution, gelation after 1 hour, some seeping-out liquid on the top after still standing for 1 day |
| | 10.4 | Transparent solution, gelation after 12 minutes, some seeping-out liquid on the top after still standing for 1 day |
| | 19.6 | Transparent solution, gelation (ringing gel) after 2 minutes |
| | 35.4 | Transparent solution, gelation (ringing gel) after 1 minute |
| Glycerin substituted silane derivative | 2.5 | Transparent solution, slight thickening after 2 hours, pasty after 24 hours (no gelation) |
| | 5.6 | Transparent solution, gelation after 3 hours, some seeping-out liquid on the top after still standing for 1 day |
| | 11.0 | Transparent solution, gelation after 1 hour, some seeping-out liquid on the top after still standing for 1 day |
| | 21.0 | Transparent solution, gelation (ringing gel) after 30 minutes |
| | 35.0 | Transparent solution, gelation (ringing gel) after 30 minutes |

As shown in Tables 2 and 3, the polyhydric alcohol substituted silane derivatives obtained in the present invention are water-soluble; therefore, the system can easily be gelated only by adding the derivative into water or into a water-based medium. In addition, it is possible to suitably adjust the hardness of the system by changing the concentration of the silane derivative to be added.
In the case of the polyhydric alcohol substituted silane derivatives obtained in the present invention, the addition and removal of acid, alkali, or an organic solvent are not necessary unlike the case of conventional water-insoluble silanized compounds (for example, tetraethoxysilane). Thus, the polyhydric alcohol substituted silane derivatives are very desirable from the viewpoint of process, cost, and environmental load.

### Blending of water-soluble metal alcoholate derivatives to external preparations

The present inventors prepared various water-soluble metal alcoholate derivatives according to the above-described synthesis examples and tried the preparation of external preparations containing the water-soluble metal alcoholate derivatives.

### Examples 14 and 15 (facial cleanser)

According to the composition in Table 4, the water-soluble silane derivative, water, and the surfactant were weighed and placed into a flat glass bottle, and the mixture was homogeneously mixed by agitation with shaking and then allowed to stand at room temperature.

**[Table 4]**

| | Example 14 | Example 15 |
|---|---|---|
| Propylene glycol substituted silane derivative Si-(OCH₂CH₂CH₂OH)₄ | 0.52 g | 0.51 g |
| Water | 0.51 g | 0.50 g |
| Sodium lauroyl glutamate (10% aq) | - | 0.50 g |

In both Examples 14 and 15, a transparent solid gel was obtained after still standing for 1 to 2 hours. In Example 15, the transparent solid gel became opaque after the subsequent still standing for long hours. In both examples, a small amount could be easily scraped with a spatula. When the obtained mass was applied on the palm and rubbed, it swiftly disintegrated and became a moist pasty powder. When the obtained mass was rubbed with a small amount of water, it spread easily on the skin surface and massaging were possible.

In Example 14, a scrubbing effect due to silica gel, which was formed by the hydrolysis and dehydration-condensation reaction of the water-soluble silane derivative, could be achieved by spreading it on the skin with a small amount of water. In addition, because of the moisturizing effect of propylene glycol, which was produced as a by-product, a moist feeling could be achieved after facial cleansing. In Example 15, adequate foaming and a facial cleansing effect were obtained, in addition to a scrubbing effect due to silica gel, because of the surfactant (acylglutamate) contained in the composition, and an after-use moist feeling could be achieved in the same way as Example 1.

### Examples 16 to 18 (facial cleanser)

According to the composition in Table 5, the water-soluble silane derivative, water, and the surfactant were weighed and placed into a flat glass bottle, and the mixture was homogeneously mixed by agitation with shaking and then allowed to stand at room temperature.

**[Table 5]**

| | Example 16 | Example 17 | Example 18 |
|---|---|---|---|
| 1,3-Butylene glycol substituted silane derivative Si-(OCH₂CH₂CH₂OHCH₃)₄ | 1.03 g | 1.92 g | 1.02 g |
| Water | 1.04 g | 0.51 g | - |
| Sodium lauroyl glutamate (10% aq) | - | 0.54 g | 1.03 g |

In Example 16, a transparent solid gel was obtained after still standing for several hours. In Example 17, an opaque solid gel was obtained after about 4 hours. In Example 18, a white cloud appeared and then solidified after about 3 hours. In all examples, a small amount could be easily scraped with a spatula. When the obtained mass was applied on the palm and rubbed, it swiftly disintegrated and became a moist pasty powder. When the obtained mass was rubbed with a small amount of water, it spread easily and a skin-like application and massaging were possible.

In Example 16, a scrubbing effect due to silica gel, which was formed by the hydrolysis and dehydration-condensation reaction of the water-soluble silane derivative, could be achieved by spreading it on the skin with a small amount of water. In addition, because of a moisturizing effect of 1,3-butylene glycol, which was produced as a by-product, a moist feeling could be achieved after facial cleansing. In Examples 17 and 18, adequate foaming and a facial cleansing effect were obtained, in addition to a scrubbing effect due to silica gel, because of the surfactant (acylglutamate) contained in the composition, and an after-use moist feeling could be achieved in the same way as Example 16.

### Example 19 (transparent whitening gel)

Into 7.6 g of deionized water, 0.2 g of tranexamic acid was dissolved. To this, 2.2 g of propylene glycol substituted silane derivative, (Si-(OCH₂CH₂CH₂OH)₄), was added, thoroughly stirred, and filled into ajar.
After the mixture was allowed to stand at room temperature, a transparent solid gel was obtained. The solid gel had extremely high transparency and had agar-like hardness; however, it was easily possible to scrape a suitable amount with a spoon applicator. When the obtained transparent solid gel was rubbed on the skin, it spread smoothly, and a moist feeling could be achieved on the skin. In addition, a whitening effect and a skin balancing effect could be achieved because of blended tranexamic acid.

The amount of the propylene glycol substituted silane derivative was decreased to 2.0 g in the above-described composition, and the mixture was mixed with stirring. Then, a slightly softer transparent gelatinous material was obtained. This material was filled into an extrusion pump-type container and allowed to stand at room temperature by the window. Even after 1 month, there was no change in the gel structure and in the extrusion easiness, and the gel was extremely stable.

### Stabilization of drug component

Subsequently, the present inventors investigated the stabilization effect when a drug component is blended in the base of a solid gelatinous external preparation that was prepared with the use of a polyhydric alcohol substituted silane derivative.

### Example 20

Into 1 g of water, 0.2 g of the propylene glycol substituted silane derivative, (Si-(OCH₂CH₂CH₂OH)₄), was dissolved. Into this solution, 0.2 mL of subtilisin solution (4 µg/mL) was added and mixed, and the mixture was allowed to stand for a fixed length of time. The obtained solid gel was crushed with a spatula and centrifuged, and a set amount of supernatant was collected. Similarly, several test samples were prepared, and they were maintained at various temperatures (0 °C, 37 °C, room temperature, and 50 °C). After 4 hours and after 3 days, the subtilisin enzyme activity of each sample was measured. The enzyme activity was measured with a fluorospectrophotometer after the reaction between Nα-p-tosyl-L-arginine methyl ester hydrochloride in a Tris-HCl buffer solution and each sample. In Comparative Example 4, the subtilisin enzyme activity was measured in the same way as Example 20 except that the propylene glycol substituted silane derivative was not used.
The values of enzyme activity were standardized by setting the enzyme activity of the comparative example sample that had been stored at 0 °C for 4 hours to 100.0. The measurement results for the above-described enzyme activity are shown in Table 6.

**[Table 6]**

| | | Comparative Example 4 | Example 20 |
|---|---|---|---|
| After 4 hours | 0 °C | 100.0 | 100.0 |
| | 37 °C | 91.5 | 109.6 |
| | 50 °C | 14.4 | 134.6 |
| After 3 days | 0°C | 59.7 | 92.3 |
| | Room temperature | 65.3 | 121.2 |
| | 37 °C | 34.9 | 115.4 |
| | 50 °C | -1.2 | 21.2 |

### Example 21

Into 1 g of water, 0.2 g of the propylene glycol substituted silane derivative, (Si-(OCH₂CH₂CH₂OH)₄), was dissolved. Into this solution, 0.2 mL of papain solution (4 µg/mL) was added and mixed, and the samples were charged on a 96-hole plate and allowed to stand for a fixed length of time. Similarly, several test samples were prepared, and they were maintained at various temperatures (0 °C, 37 °C, room temperature, and 50 °C). After 2 hours and after 3 days, the papain enzyme activity of each sample was measured. The enzyme activity was measured with a plate reader after the enzyme substrate, N-carbobenzoxy-phenylarginine-4-methylcoumarinamide (Z-PheArg-MCA), was directly added into the 96-hole plate and allowed to stand for 20 minutes at room temperature. In Comparative Example 5, the papain enzyme activity was measured in the same way as Example 21 except that the propylene glycol substituted silane derivative was not used.
The values of enzyme activity were standardized by setting the enzyme activity of the comparative example sample that had been stored at 0 °C for 2 hours to 100.0. The measurement results for the above-described enzyme activity are shown in Table 7.

**[Table 7]**

| | | Comparative Example 5 | Example 21 |
|---|---|---|---|
| After 2 hours | 0 °C | 100.0 | 100.0 |
| | Room temperature | 96.5 | 141.7 |
| | 37 °C | 98.3 | 139.0 |
| | 50 °C | 67.1 | 135.0 |
| After 3 days | 0°C | 98.9 | 90.3 |
| | Room temperature | 34.5 | 111.4 |
| | 37 °C | 18.6 | 115.5 |
| | 50 °C | 2.2 | 53.0 |

A protease is blended into cosmetics for skin improvement by the promotion of stratum corneum desquamation. However, as is clear from the above-described comparative examples, a protease is deactivated in an aqueous solution by high temperature or with the elapse of time. Thus, it is usually necessary to prepare a formulation with dry state powder, or it is necessary to try special immobilization such as a glutaraldehyde treatment in order to retain the activity of a hydrous formulation.
On the other hand, as shown in Examples 20 and 21, high enzyme activity can be retained in the solid gelatinous external preparation of the present invention at a high temperature or with the elapse of time. Thus, the practical-level enzyme activity can be retained under the normal storage conditions of cosmetics, and the effect of stratum corneum desquamation at the time of production can be retained for a long term.

### Example 22: Solid gelatinous makeup remover

**[Table 8]**

| | Weight % |
|---|---|
| Propylene glycol substituted silane derivative Si-(OCH₂CH₂CH₂OH)₄ | 15 |
| Polyoxyethylene (8 mol) glyceryl monoisostearate | 20 |
| Decamethylcyclopentasiloxane | 8 |
| Isopropyl myristate | 20 |
| Purified water | balance |

The solid gelatinous makeup remover obtained in Example 22 was a white gel, it swiftly disintegrated and smoothly spread on the skin, and a scrubbing effect due to silica gel and a moist feeling due to polyhydric alcohol could be achieved.

### Example 23: Protease-containing solid gelatinous scrubbing agent

**[Table 9]**

| | Weight % |
|---|---|
| 1,3-Butylene glycol substituted silane derivative Si-(OCH₂CH₂CH₂OHCH₃)₄ | 35 |
| Protease (subtilisin) | 3 |
| Citric acid | 0.05 |
| Sodium citrate | 0.05 |
| Phenoxyethanol | q.s. |
| Ethanol | 8 |
| Purified water | balance |

The protease-containing solid gelatinous scrubbing agent obtained in Example 23 swiftly disintegrated and smoothly spread on the skin, and a scrubbing effect due to silica gel and a moist feeling due to polyhydric alcohol could be achieved.

### Example 24: Protease-containing solid gelatinous cosmetic

**[Table 10]**

| | Weight % |
|---|---|
| 1,3-Butylene glycol substituted silane derivative Si-(OCH₂CH₂CH₂OHCH₃)₄ | 20 |
| Protease (subtilisin) | 2.5 |
| Glycerin | 2 |
| 1,3-Butylene glycol | 5 |
| Citric acid | 0.05 |
| Sodium citrate | 0.05 |
| Phenoxyethanol | q.s. |
| Ethanol | 5 |
| Purified water | balance |

The protease-containing solid gelatinous cosmetic obtained in Example 24 swiftly disintegrated and smoothly spread on the skin, and a scrubbing effect due to silica gel and a moist feeling due to polyhydric alcohol could be achieved.

### Example 25: Solid gelatinous moisturizing foundation

**[Table 11]**

| | Weight % |
|---|---|
| 1,3-Butylene glycol substituted silane derivative Si-(OCH₂CH₂CH₂OHCH₃)₄ | 12 |
| Dimethylpolysiloxane | 15 |
| Decamethylcyclopentasiloxane | 2 |
| 1,3-Butylene glycol | 6 |
| Candelilla wax | 3 |
| Titanium oxide | 7.5 |
| Barium sulfate | 5 |
| Titanium oxide | 7 |
| Talc | 3 |
| Silicic anhydride | 4 |
| Sodium metaphosphate | 0.1 |
| Sodium hyaluronate | 0.1 |
| p-Oxybenzoic acid ester | q.s. |
| Red iron oxide | q.s. |
| Yellow iron oxide | q.s. |
| Black iron oxide | q.s. |
| Xanthan gum | 0.2 |
| Sodium carboxymethylcellulose | 0.2 |
| Purified water | balance |

The solid gelatinous moisturizing foundation obtained in Example 25 swiftly disintegrated and smoothly spread on the skin, and a moist feeling due to polyhydric alcohol could be achieved.

### Example 26: Solid gelatinous nutrient cream

**[Table 12]**

| | Weight % |
|---|---|
| 1,3-Butylene glycol substituted silane derivative Si-(OCH₂CH₂CH₂OHCH₃)₄ | 10 |
| Liquid paraffin | 8 |
| Petrolatum | 3 |
| Dimethylpolysiloxane | 2 |
| Stearyl alcohol | 3 |
| Behenyl alcohol | 2 |
| Glycerin | 5 |
| Dipropylene glycol | 4 |
| Trehalose | 1 |
| Pentaerythritol tetra-2-ethylhexanoate | 4 |
| Polyoxyethylene glyceryl monoisostearate | 2 |
| Polyoxyethylene glyceryl monostearate | 1 |
| Lipophilic glyceryl monostearate | 2 |
| Citric acid | 0.05 |
| Sodium citrate | 0.05 |
| Potassium hydroxide | 0.015 |
| Oil-soluble licorice extract | 0.1 |
| Tocopherol acetate | 0.1 |
| p-Oxybenzoic acid ester | q.s. |
| Phenoxyethanol | q.s. |
| Dibutylhydroxytoluene | q.s. |
| Trisodium edetate | 0.05 |
| 4-t-Butyl-4'-methoxydibenzoylmethane | 0.01 |
| 2-Ethylhexyl p-methoxycinnamate | 0.1 |
| Hydroxyethyl cellulose | 0.5 |
| Perfume | q.s. |
| Purified water | balance |

The solid gelatinous nutrient cream obtained in Example 26 swiftly disintegrated and smoothly spread on the skin, and a scrubbing effect due to silica gel and a moist feeling due to polyhydric alcohol could be achieved.

### Example 27: Solid gelatinous massage cream

**[Table 13]**

| | Weight % |
|---|---|
| Propylene glycol substituted silane derivative Si-(OCH₂CH₂CH₂OH)₄ | 8 |
| Liquid paraffin | 35 |
| Petrolatum | 10 |
| Microcrystalline wax | 1 |
| Cetanol | 2 |
| Batyl alcohol | 1 |
| Glycerin | 3 |
| Dipropylene glycol | 6 |
| Squalane | 6 |
| Stearic acid | 1 |
| Cetyl 2-ethylhexanoate | 10 |
| Polyoxyethylene hydrogenated castor oil | 1.5 |
| Polyoxyethylene hydrogenated castor oil pyroglutamate isostearate | 0.1 |
| Polyoxyethylene glyceryl monostearate | 1 |

| | |
|---|---|
| Potassium hydroxide | 0.1 |
| Sodium metaphosphate | 0.02 |
| L-arginine | 0.1 |
| Perfume | q.s. |
| Purified water | balance |

The solid gelatinous massage cream obtained in Example 27 swiftly disintegrated and smoothly spread on the skin, and a scrubbing effect due to silica gel and a moist feeling due to polyhydric alcohol could be achieved.

### Example 28 (facial cleanser)

According to the composition in Table 14, the water-soluble silane derivative, water, and the surfactant were weighed and placed into a flat glass bottle, and the mixture was homogeneously mixed by agitation with shaking and then allowed to stand at room temperature.

**[Table 14]**

| | Weight % |
|---|---|
| Ethylene glycol substituted silane derivative Si-(OCH₂CH₂OH)₄ | 50 |
| Ion-exchanged water | 28 |
| POE lauryl ether sulfate | 5 |
| Imidazolium betaine | 11 |
| Glyceryl mono-2-ethylhexanoate | 6 |

In Example 28, a white solid gel was obtained immediately after mixing. The facial cleanser obtained in Example 15 could be easily scraped in a small amount with a spatula. When the facial cleanser was applied on the palm and rubbed, it swiftly disintegrated and became a moist pasty powder. When the facial cleanser was rubbed with a small amount of water, it spread easily on the skin surface and massaging were possible.
When lipstick was applied on the back of the hand and a small amount of the facial cleanser obtained in Example 28 was applied on it and rubbed, the lipstick swiftly disappeared and a moist after-use feeling could be achieved.

## Claims

1. A water-soluble metal alcoholate derivatives represented by the following general formula (1).
M¹-(OR¹)ₙ (1)
(In the formula, M¹ represents a Si, Ti, Zr, Zn, or Al atom, and R¹ represents a polyhydric alcohol residue. When M¹ is a Si, Ti or Zr atom, n is 4, when M¹ is a Zn atom n is 2, and when M¹ is an Al atom, n is 3.)

2. The water-soluble metal alcoholate derivative according to claim 1, wherein the M¹ in formula (1) is Si or Ti atom.

3. The water-soluble metal alcoholate derivative according to claim 1 or 2, wherein the R¹ in formula (1) is any one selected from the group consisting of an ethylene glycol residue, a propylene glycol residue, a butylene glycol residue, and a glycerin residue.

4. A production method of water-soluble metal alcoholate derivatives comprising reacting a metal alkoxide and a polyhydric alcohol under the presence of a solid catalyst.

5. The production method of water-soluble metal alcoholate derivatives according to claim 4, wherein the metal alkoxide is titanium alkoxide or alkoxysilane.

6. The production method of water-soluble metal alcoholate derivatives according to claim 4 or 5, wherein the reaction is carried out under the temperature condition of 5 to 90 °C.

7. The production method of water-soluble metal alcoholate derivatives according to any one of claim 4 to 6, wherein the solid catalyst is an ion exchange resin.

8. The production method of water-soluble metal alcoholate derivatives according to any one of claim 4 to 7, wherein the metal alkoxide is tetraethoxysilane.

9. The production method of water-soluble metal alcoholate derivatives according to any one of claim 4 to 8, wherein the polyhydric alcohol is any one selected from the group consisting of ethylene glycol, propylene glycol, butylene glycol, and glycerin.

10. The production method of water-soluble metal alcoholate derivatives according to any one of claim 4 to 9, wherein the polyhydric alcohol is any one selected from the group consisting of ethylene glycol, propylene glycol, and butylene glycol, and that the reaction is carried out under the temperature condition of 5 to 35 °C.

11. A solid gelatinous external preparation obtained by blending with the water-soluble metal alcoholate derivative according to any one of claim 1 to 3 and water.

12. The solid gelatinous external preparation according to claim 11, further blended with a drug component.

13. A production method of the solid gelatinous external preparation comprising, adding the water-soluble metal alcoholate derivative according to any one of claim 1 to 3 into a formulation of external preparation containing water.

14. A production method of the solid gelatinous external preparation comprising, mixing the water-soluble metal alcoholate derivative according to any one of claim 1 to 3 and water to prepare a solid gel, and adding the obtained solid gel to a formulation of external preparation.
